# EUROPEAN PATENT APPLICATION

(11) **EP 4 786 002 A2**
(43) Date of publication of application: **05.08.2026**
(21) Application number: 26186022.5
(22) Date of filing: 29.03.2022
(51) Int. Cl.: A61P 31/04

(54) **IMMUNOGENIC FUSION PROTEIN**

(30) Priority: 29.03.2021 EP 21165674
(62) Divisional of application: 22713429.3
(71) Applicant: MinervaX ApS, 2000 Frederiksberg (DK)
(72) Inventor: FISCHER, Per, 2000 Frederiksberg (DK)
(74) Representative: Öberg & Sjöberg AB

(57) **Abstract**

The present invention relates to a vaccine comprising: a) an immunogenic fusion protein consisting of amino acids 2-347 of SEQ ID NO. 21; and b) a further immunogenic fusion protein consisting of amino acids 2-347 of SEQ ID NO: 42. The invention further pertains to the vaccine for use as a medicament and in i) vaccination against group B Streptococcus infection or preventing a group B Streptococcus infection, or ii) treating a group B Streptococcus infection.

## Description

### FIELD OF INVENTION

The present invention relates to the fields of microbiology and vaccine technology and concerns the development of an immunogenic fusion protein capable of conferring immunity to group B Streptococcus infections. More particularly, the present invention relates to an immunogenic fusion protein which confers immunity to invasive strains of the group B Streptococcus. It further pertains to a nucleic acid molecule encoding the immunogenic fusion protein; a vector; a host cell; a vaccine; and a method of vaccination against group B Streptococcus infection or treating a group B Streptococcus infection.

### BACKGROUND OF THE INVENTION

Group B Streptococcus (*Streptococcus agalactiae)* (GBS) is the major cause of invasive bacterial infections, including meningitis, in the neonatal period. In the United States alone, there are now about 5000 cases per year of invasive disease caused by this bacterium. These infections have an overall mortality of about 10%, and many of the infants that survive have permanent neurological sequelae. In view of this, a large effort has been made to find methods of prevention and treatment and to analyze the mechanisms by which GBS cause infections.

GBS can also cause mastitis in cows, a bovine disease that is of considerable economical importance. Development of a vaccine against GBS infections is therefore of interest also in veterinary medicine.

About 20 % of all women are vaginal carriers of GBS, and vertical transmission from the maternal genital tract is probably the most common source of infection in neonatal disease caused by this bacterium. However, only about 1 % of the infants that are colonized by the GBS at birth are afflicted by serious infection. Other factors than exposure to the bacterium during birth must therefore contribute to the development of neonatal disease.

Group B streptococcal strains are divided into nine serotypes (la, Ib, and II-VIII) based on the structure of the polysaccharide capsule. The four "classical" serotypes Ia, Ib, II, and III occur in roughly equal proportions among strains in the normal flora, but type III is the clinically most important serotype, in particular because it causes most cases of meningitis. Because the capsule is a known virulence factor, it has been studied in considerable detail, in particular in type III strains. Efforts have been made to develop a vaccine, in which the type III polysaccharide capsule would be an essential component.

EP 0 866 133 discloses a vaccine capable of protecting a recipient from infection caused by group B *Streptococcus.* The invention is directed to the use of a combination of a polysaccharide and a fragment of the epsilon protein. It further discloses that epidemiological data suggest that the type-specific capsule plays an important role in the immunity to group B *Streptococcus* infections (see page 7 line 2-3). Additionally, there are a number of different combinations between different proteins and the polysaccharide mentioned within the application, but all the claims comprise a polysaccharide which shows the importance of that particular component. However, use of the polysaccharide capsule as a vaccine may give problems due to cross reactions with human tissues (Pritchard et al., Infect Immun 1992. 60: 1598). It would therefore be very valuable if one could develop a vaccine based on proteins rather than on polysaccharides.

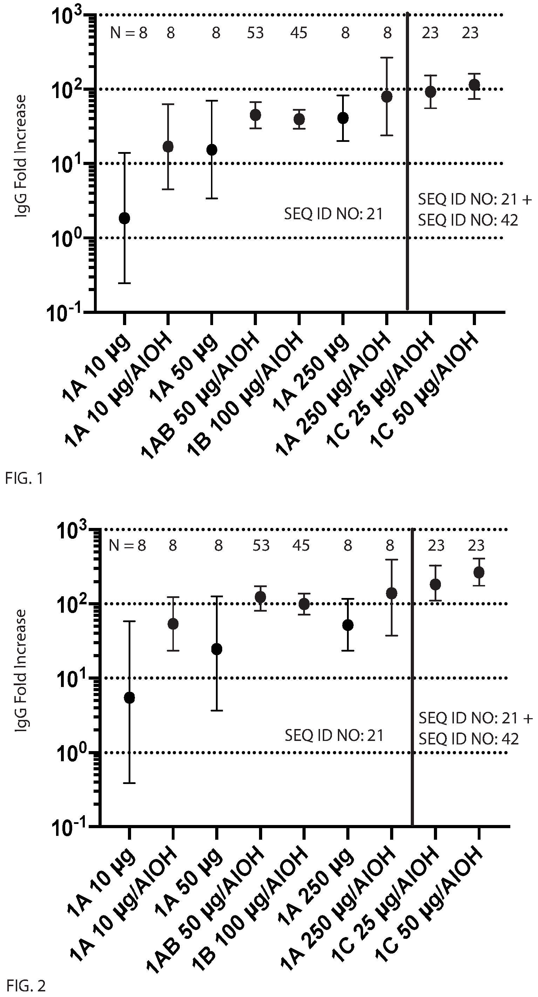

The document Gravekamp et al., Infection and Immunity, Dec 1997, p 5216-5221 discloses the evaluation of the immunogenicity as well as protection of the number of repeats of the alpha (α) C protein as well as the N-terminal part alone. It was found that the immunogenicity decreased with increasing number of repeats (se Fig 2B). However, it was also found in a protection assay that the antibodies against the N-terminal region were predominantly responsible for the protection compared to antibodies against the N-terminal region (see page 5219 left column, line 6 from the bottom, and page 5220 right column lines 26-29).

WO 9410317 describes the use of the alpha protein, a GBS surface protein, in the development of a conjugate vaccine. A drawback with this protein is that it usually is not expressed by type III strains, which are the cause of many serious GBS infections. Hence, a protective immunity against these strains will not be evoked by an alpha protein vaccine.

WO 9421685 describes the use of the Rib protein, a GBS surface protein, in the development of a vaccine. This protein elicits immunity when administered with alum. However, the Rib protein has the disadvantage that it does not evoke a protective immunity against all GBS strains.

The document Lindahl et al, Nonimmunodominant Regions Are Effective As Building Blocks In A Streptococcal Fusion Protein Vaccine, Cell Host & Microbe 2, 427-434, December 2007, discloses a fusion protein comprising N-terminal regions of the group B Streptococcus surface proteins Rib and AlpC.

Similarly, WO 2008127179 describes a fusion protein comprising at least one first N-terminal region fragment of a group B Streptococcus surface protein or analogue, homologue, derivative or immunologically related amino acid sequence or fragments thereof, which is fused to at least one second N-terminal region fragment of a group B Streptococcus surface protein or analogue, homologue, derivative or immunologically related amino acid sequence or fragments thereof, wherein the first and second at least one N-terminal region fragments of group B Streptococcus surface proteins derive from different group B Streptococcus strains, and wherein the fusion protein is capable of eliciting protective immunity against group B Streptococcus.

Despite the advances in the progress towards a vaccine suitable for prevention of GBS disease, there is still a need for further methods and vaccines for prevention and treatment of GBS infections. Thus, there remains a need to explore vaccines strategies capable of eliciting protective immunity against a wide range of GBS strains.

Accordingly, it is a primary objective of the present invention to provide an immunogenic fusion protein which can be used in a vaccine capable of eliciting protective immunity against GBS infections.

It is a further objective of the present invention to provide a vaccine that elicits protective immunity against many clinically important GBS strains.

Another objective of the present invention is to provide a vaccine comprising a single, or a few, immunogenic fusion proteins that elicits protective immunity against GBS infections. A single or a few proteins has several advantages over a vaccine composed of numerous proteins, e.g., cost of production and safety.

It is a further objective of the present invention to provide an immunogenic fusion protein capable of providing improved protection against GBS.

The means of accomplishing each of the above objectives as well as others will become apparent from the description of the invention which follows hereafter.

### SUMMARY OF THE INVENTION

The present invention is based on further work, by the present inventor, on the fusion protein disclosed in WO 2008127179. Specifically, an immunogenic fusion protein with a shorter amino acid sequence of the N terminal regions of Rib and AlpC has been found to be suitable. Such an immunogenic fusion protein may be easier to produce and use for vaccination. Further, as shown in Examples 4 and 5, such a shorter fusion protein will provide improved protection against GBS, specifically by providing improved protection against adhesion and/or invasion of endothelial cells.

Additionally, codon optimized corresponding nucleic acid molecules encoding the immunogenic fusion protein have been devised. These may be provided in a vector. A host cell may in turn express the immunogenic fusion protein or comprise the vector. Each of the immunogenic fusion protein, the nucleic acid molecule, the vector, and the host cell may be used in a vaccine. The immunogenic fusion protein, the nucleic acid molecule, the vector, the host cell, and/or the vaccine may further be used in a method of vaccination against group B Streptococcus infection or treating a group B Streptococcus infection.

Thus, a first aspect of the present invention relates to an immunogenic fusion protein comprising or consisting of an amino acid sequence consisting of:
i. a first amino acid sequence part consisting of 170 to 178 amino acids, preferably 174 to 175 amino acids, and having at least 90% sequence identity with the amino acid sequence shown in SEQ ID NO: 7;
ii. a second amino acid sequence part consisting of 165 to 174 amino acids, preferably 169 to 170 amino acids, and having at least 90% sequence identity with the amino acid sequence shown in SEQ ID NO: 14; and optionally:
iii. a linker amino acid sequence part consisting of 1 to 20 amino acids and separating the first amino acid sequence part from the second amino acid part,
and wherein the immunogenic fusion protein preferably consists of 335 to 372 amino acids, preferably 343 to 353 amino acids, more preferably 343 to 347 amino acids.

As noted above, the immunogenic fusion protein may be easier to produce and use for vaccination. The immunogenic fusion protein has the advantage that it is immunogenic even without adjuvant, however it can also be used with an adjuvant such as alum or Aluminum hydroxide (AlOH) for increased immunogenicity.

Another advantage with the immunogenic fusion protein is that it can be combined with a further immunogenic fusion protein comprising the N-terminal regions of Alp1 and Alp2. As seen in the examples, the combination of two such immunogenic fusion proteins provide a synergistic effect on the numbers of antibodies (antibody titers) that are obtained when the combination is used for immunization.

The present invention will be described in more detail below.

### BRIEF DESCRIPTION OF THE FIGURES

Fig. 1 shows IgG fold increase obtained 29 days after immunization using one dose of either an immunogenic fusion protein according to the first aspect of the present invention alone, or in combination with a further immunogenic fusion protein as encompassed by the vaccine according to the fifth aspect of the present invention, at different dosages with or without an adjuvant.
Fig. 2 shows IgG fold increase obtained 57 days from immunization using a first dose and a second dose of either an immunogenic fusion protein according to the first aspect of the present invention alone, or in combination with a further immunogenic fusion protein as encompassed by the vaccine according to the fifth aspect of the present invention, at different dosages with or without an adjuvant.
Fig. 3 shows survival of mice pups following neonatal bacterial infection with group B Streptococcus strain BM110 (8 x 10³ cfu). The pups were born from female mice having been immunized with either a) the immunogenic fusion protein of SEQ ID NO: 21 together with the adjuvant aluminum hydroxide (AlOH), b) or control (only the adjuvant aluminum hydroxide).
Fig. 4A-4E show results obtained in a human epithelial cell invasion study using clinical isolates and antisera obtained from immunization using the immunogenic fusion protein of SEQ ID NO: 21.
Figs. 5A-5Cshow representations of the fusion protein according to WO2008127179 including the leading sequence according to SEQ ID NO: 43 and the shorter amino acid sequence of the fusion protein according to SEQ ID NO: 21.
Fig. 6 shows the interaction between the shorter fusion protein of SEQ ID NO: 21 and α1β1-integrin.

### DETAILED DESCRIPTION OF THE INVENTION

In this specification, unless otherwise specified, "a" or "an" means "one or more". Throughout the specification, any and all references are specifically incorporated into this patent application by reference.

As above, a first aspect of the present invention relates to an immunogenic fusion protein comprising or consisting of an amino acid sequence consisting of:
i. a first amino acid sequence part consisting of 170 to 178 amino acids, preferably 174 to 175 amino acids, and having at least 90% sequence identity with the amino acid sequence shown in SEQ ID NO: 7;
ii. a second amino acid sequence part consisting of 165 to 174 amino acids, preferably 169 to 170 amino acids, and having at least 90% sequence identity with the amino acid sequence shown in SEQ ID NO: 14; and optionally:
iii. a linker amino acid sequence part consisting of 1 to 20 amino acids and separating the first amino acid sequence part from the second amino acid part,
and wherein the immunogenic fusion protein preferably consists of 335 to 372 amino acids, preferably 343 to 353 amino acids, more preferably 343 to 347 amino acids.

The term "immunogenic" is intended to mean having the ability to elicit an immune response. The immunogenic fusion protein of the invention is immunogenic and characterized by its ability to elicit a protective immune response against at least GBS containing the surface proteins of which the N-terminal regions are comprised by the immunogenic fusion protein. Eliciting protective immunity against group B Streptococcus is to be understood as encompassing eliciting protective immunity against group B streptococcus carrying at least one of the surface proteins Rib and AlpC. "Capable" means that the immunogenic fusion protein, if administered in a suitable dose to an individual having an immune system, elicits protective immunity in that individual. The immunogenic fusion protein is preferably capable of eliciting protective immunity against group B Streptococcus.

The term "protective immunity" in relation to the present invention refers to the ability of serum antibodies and/or cytotoxic T cell response induced during immunization to protect (partially or totally) against disease caused by an infectious agent, such as a group B Streptococcus. That is, a vertebrate immunized by the vaccines of the invention will experience limited growth and spread of group B Streptococcus. To determine whether protective immunity is induced by a fusion protein or vaccine, techniques well known for a person skilled in the art can be used. For example, to determine whether immunization with a fusion protein or vaccine of the invention induces protective immunity against group B Streptococcus infection, immunized test animals can be challenged with group B Streptococcus and growth and spread of the group B Streptococcus is measured. For example, to determine whether protective immunity is induced, methods in accordance with the methods described in the examples below can be used.

For the purpose of the present invention the term "fusion protein" refers to an assembly of two or more protein regions, or fragments thereof. As stated above, the immunogenic fusion protein comprises (or preferably consists) of an amino acid sequence consisting of at least first and second amino acid sequence parts. These parts are related to the N-terminal regions of the group B Streptococcus surface proteins Rib and AlpC, respectively.

The combination of amino acid sequence parts to obtain a fusion protein can be accomplished by fusing, i.e., joining, the amino acid sequence parts together at one or both ends thereof (covalent coupling, bond, or linking, or conjugation), or by joining together separate nucleic acid molecules encoding the respective amino acid sequence parts, such that a single continuous expression product is produced when the joined nucleic acid molecules are translated.

In a fusion protein, a first and a second amino acid sequence part may be separated by a linker amino acid sequence part.

It is further contemplated within the context of the present invention that a fusion protein can be obtained by cross-linking the amino acid sequence parts or by capturing them on a macromolecular structure.

For the purpose of the present invention the term "protein" refers to a molecular chain of amino acids. A protein is not of a specific length and can, if required, be modified in vivo or in vitro, by, for example, glycosylation, amidation, carboxylation or phosphorylation. Inter alia, peptides, oligopeptides and polypeptides are included within the definition.

The term "N-terminal region" in relation to the present invention refers to an N-terminus region (N) of a protein.

Group B streptococcus strains, also referred herein as GBS, are known and may be isolated from the blood of infected human beings. GBS is the most common cause of neonatal sepsis in the United States and is responsible for about 5000 cases per year.

The denotation "Group B streptococcus" derives from the fact that Streptococci have been divided into immunological groups based upon the presence of specific carbohydrate antigens on their cell surfaces. At present, groups A through O are recognized.

The group B Streptococcus Rib protein, also referred to in this specification as Rib and Rib protein, is a surface protein known in the art, and for example described in WO 9421685. The denotation "Rib" refers to: Resistance to proteases, immunity, and group B. The Rib protein was first isolated from a group B streptococcal strain of serotype III as a distinct 95 kDa protein. Protein Rib is expressed by almost all group B streptococcal strains of the clinically important serotype III, which cause most cases of meningitis, and by some strains of other serotypes such as II. Moreover, Rib is expressed by all strains of a hypervirulent clone of type III. A method has been devised to purify protein Rib and it has been demonstrated that antibodies to this protein protect against lethal infection with strains expressing protein Rib (for further details, such as DNA and protein sequences see WO 9421685).

SEQ ID NO: 7 shows the shorter N-terminal Region of Rib:

MAEVISGSAVTLNTNMTKNVQNGRAYIDLYDVKNGKIDPLQLITLNSPDLK
AQYVIRQGGNYFTQPSELTTVGAASINYTVLKTDGSPHTKPDGQVDIINVSL
TIYNSSALRDKIDEVKKKAEDPKWDEGSRDKVLISLDDIKTDIDNNPKTQSD
IANKITEVTNLEKILVPRIP

Nucleic acid DNA molecules encoding the amino acid sequence of SEQ ID NO: 7 are shown in SEQ ID NOs: 1, 3, 5, where SEQ ID NO: 3 is codon optimized for expression in *E. coli,* and SEQ ID NO: 5 is codon optimized for expression in *Cricetulus griseus* (Chinese Hamster Ovary) cells.

Corresponding nucleic acid RNA molecules encoding the amino acid sequence of SEQ ID NO: 7 are shown in SEQ ID NOs: 2, 4, 6, where SEQ ID NO: 4 is codon optimized for expression in *E. coli,* and SEQ ID NO: 6 is codon optimized for expression in *Cricetulus griseus* (Chinese Hamster Ovary) cells.

The difference between the shorter N-terminal region of Rib, i.e. SEQ ID NO: 7, and the sequence given for the N-terminal of Rib in WO 2008127179 is the 5 amino acid long leading sequence GPLGS, SEQ ID NO: 44.

The group B Streptococcus AlpC protein, also known as alpha protein, is a group B Streptococcus surface protein known in the art. WO 9410317 describes a conjugate vaccine composition comprising the alpha protein. The native group B Streptococcus AlpC precursor protein as described in WO 9410317 has a molecular weight of 108 kDa. Cleavage of the putative signal sequence of 41 amino acids yields a mature protein of 104 kDa. (Note, however, that the signal sequence was subsequently shown to have a length of 56 amino acid residues: Stålhammar-Carlemalm et al., J Exp Med 177,1593; 1993). The 20 kDa N-terminal region of the AlpC antigen shows no homology to previously described protein sequences and is followed by a series of nine tandem repeating units that make up 74% of the mature protein. Each repeating unit (denoted herein as "R") is identical and consists of 82 amino acids with a molecular mass of about 8500 Daltons, which is encoded by 246 nucleotides. The C-terminal region of the AlpC antigen contains a cell wall anchor domain motif present in a number of gram-positive surface proteins.

SEQ ID NO: 14 shows the shorter N-terminal Region of AlpC:

STIPGSAATLNTSITKNIQNGNAYIDLYDVKLGKIDPLQLIVLEQGFTAKYVF
RQGTKYYGDVSQLQSTGRASLTYNIFGEDGLPHVKTDGQIDIVSVALTIYDS
TTLRDKIEEVRTNANDPKWTEESRTEVLTGLDTIKTDIDNNPKTQTDIDSKIV
EVNELEKLLVLS

The difference between the shorter N-terminal region of AlpC, i.e. SEQ ID NO: 14, and the sequence given for the N-terminal of AlpC in WO 2008127179 is the 5 amino acid long leading sequence GPLGS, SEQ ID NO: 44.

Nucleic acid DNA molecules encoding the amino acid sequence of SEQ ID NO: 14 are shown in SEQ ID NOs: 8, 10, 12, where SEQ ID NO: 10 is codon optimized for expression in *E. coli,* and SEQ ID NO: 12 is codon optimized for expression in *Cricetulus griseus* (Chinese Hamster Ovary) cells.

Corresponding nucleic acid RNA molecules encoding the amino acid sequence of SEQ ID NO: 14 are shown in SEQ ID NOs: 9, 11, 13, where SEQ ID NO: 11 is codon optimized for expression in *E. coli,* and SEQ ID NO: 13 is codon optimized for expression in *Cricetulus griseus* (Chinese Hamster Ovary) cells.

The term "sequence identity" indicates a quantitative measure of the degree of homology between two amino acid sequences of equal length or between two nucleotide sequences of equal length. If the two sequences to be compared are not of equal length, they must be aligned to best possible fit. Sequence identity can, for example, be calculated by the BLAST program e.g., the BLASTP program or the BLASTN program (Pearson W. R and D. J. Lipman (1988) PNAS USA 85:2444-2448) (www.ncbl.nlm.nlh.gov/BLAST).

Preferably the immunogenic fusion protein consists of the amino acid sequences. The first amino acid sequence part consisting of 170 to 178 amino acids, preferably 174 to 175 amino acids, has at least 90% sequence identity with the full length of the amino acid sequence shown in SEQ ID NO: 7.

The second amino acid sequence part consisting of 165 to 174 amino acids, preferably 169 to 170 amino acids, has at least 90% sequence identity with the full length of the amino acid sequence shown in SEQ ID NO: 14.

The first amino acid sequence part should consist of at the most 178 amino acids, such as 170 to 178 amino acids, preferably 174 to 175 amino acids. As noted, SEQ ID NO: 7 contains 175 amino acids. The first M in the sequence is optional, thus resulting in 174 amino acids in these cases.

The first amino acid sequence part has at least 90%, such as at least 95%, such as at least 97%, at least 98%, at least 99%, or 100% sequence identity with the amino acid sequence shown in SEQ ID NO: 7.

The second amino acid sequence part should consist of at the most 174 amino acids, such as 165 to 174 amino acids, preferably 169 to 170 amino acids. As noted, SEQ ID NO: 14 contains 170 amino acids. The first A in the sequence may be optional, thus resulting in 169 amino acids in these cases.

The second amino acid sequence part has at least 90%, such as at least 95%, such as at least 97%, at least 98%, at least 99%, or 100% sequence identity with the amino acid sequence shown in SEQ ID NO: 14.

The linker amino acid sequence part is optional. In other words, the linker amino acid sequence part, if included, may consist of 0 to 20 amino acids. The amino acids in the linker amino acid sequence part may be selected randomly, i.e., to obtain a random coil formation. The linker amino acid sequence part, if present, separates the first amino acid sequence part from the second amino acid part. In other words, the linker amino acid sequence part is provided between the first amino acid sequence part from the second amino acid part.

Either of the first and second amino acid sequence parts may be provided with a leading/trailing amino acid sequence part at the end furthest away from the other of the first and second amino acid sequence parts. The leading/trailing amino acid sequence part may comprise one or more amino acids.

The immunogenic fusion protein preferably consists of 335 to 372 amino acids, preferably 343 to 353 amino acids, more preferably 343 to 347 amino acids.

In a preferred embodiment of the immunogenic fusion protein according to the first aspect of the present invention:
- the first amino acid sequence part comprises, or preferably consists of, amino acids 1-175 or 2-175 of the sequence shown in SEQ ID NO: 7, and
- the second amino acid sequence part comprises, or preferably consists of, amino acids 1-170 or 2-170 of the sequence shown in SEQ DI NO: 14.

SEQ ID NO: 7 contains 175 amino acids. Amino acids 2-175 correspond to the sequence of amino acids in SEQ ID NO: 7 except for the first methionine (M)
In a preferred embodiment of the immunogenic fusion protein according to the first aspect of the present invention, the amino acid sequence comprises the linker amino acid sequence part, and the linker amino acid sequence part consists of 1 to 10 amino acids, preferably 2 to 5 amino acids, more preferably 2 amino acids, and most preferably the amino acid sequence EF.

When the linker amino acid sequence part consists of the amino acids EF (Glutamate-Phenylalanine), then it is preferable that the second amino acid sequence part comprises, or preferably consists of, amino acids 2-170 of the sequence shown in SEQ DI NO: 14.

Preferably the immunogenic fusion protein has at least 90% sequence identity with, preferably all of, the amino acid sequence shown in SEQ ID NO: 21.

In a preferred embodiment the immunogenic fusion protein consists of the sequence of amino acids shown in SEQ ID NO: 21, or alternatively, the immunogenic fusion protein consists of the amino acids 2-347 of SEQ ID NO: 21.

As shown in relation to the figures, such an immunogenic fusion protein is shorter than the immunogenic fusion protein disclosed in WO 2008127179 yet has a good effect and is suitable to use in a vaccine against GBS.

Further, as shown in examples 4 and 5, such a shorter amino acid sequence, by the removal of the leading sequence SEQ ID NO: 43, will be more efficient and improved in providing protection against GBS, specifically by providing more efficient and/or improved protection against adhesion and/or invasion of endothelial cells.

SEQ ID NO: 21 corresponds to the amino acid sequence:

MAEVISGSAVTLNTNMTKNVQNGRAYIDLYDVKNGKIDPLQLITLNSPDLK
AQYVIRQGGNYFTQPSELTTVGAASINYTVLKTDGSPHTKPDGQVDIINVSL
TIYNSSALRDKIDEVKKKAEDPKWDEGSRDKVLISLDDIKTDIDNNPKTQSD
IANKITEVTNLEKILVPRIPEFSTIPGSAATLNTSITKNIQNGNAYIDLYDVKL
GKIDPLQLIVLEQGFTAKYVFRQGTKYYGDVSQLQSTGRASLTYNIFGEDGL
PHVKTDGQIDIVSVALTIYDSTTLRDKIEEVRTNANDPKWTEESRTEVLTGL
DTIKTDIDNNPKTQTDIDSKIVEVNELEKLLVLS

The difference between the shorter fusion protein according to SEQ ID NO: 21 and the sequence given for the fusion protein of WO 2008127179 is the 32 amino acid long leading sequence: GPLGSASVLIGISFLGGFTQGQFNISTDTVFA - SEQ ID NO: 43, which sequence is not found in SEQ ID NO: 21.

In a further embodiment of the immunogenic fusion protein according to the first aspect of the present invention the immunogenic fusion protein is modified by glycosylation, amidation, carboxylation or phosphorylation, or by being conjugated to a capsular polysaccharide or an RSV antigen as described with regard to the third aspect of the present invention further below.

This is advantageous as such fusion proteins may have enhanced immunogenicity. Numerous techniques are available and well known to those of skill in the art which may be used, without undue experimentation, to substantially increase the immunogenicity of the fusion protein. For example, the fusion protein may be modified by coupling to dinitrophenol groups or arsanilic acid, or by denaturation with heat and/or SDS. Alternatively, the fusion protein may be coupled to an immunogenic carrier. For a review of some general considerations in coupling strategies, see Antibodies, A Laboratory Manual, Cold Spring Harbor Laboratory, ed. E. Harlow and D. Lane (1988). Useful immunogenic carriers are well known in the art. Examples of such carriers are keyhole limpet hemocyanin (KLH); albumins such as bovine serum albumin (BSA) and ovalbumin, PPD (purified protein derivative of tuberculin); red blood cells; tetanus toxoid; cholera toxoid; agarose beads; activated carbon; or bentonite.

In a further embodiment the immunogenic fusion protein is conjugated to a capsular polysaccharide, preferably a bacterial polysaccharide, more preferably a group B Streptococcus polysaccharide.

By polysaccharide is meant any linear or branched polymer consisting of monosaccharide residues, usually linked by glycosidic linkages, and thus includes oligosaccharides. Preferably, the polysaccharide will contain between 2 and 50 monosaccharide unites, more preferably between 6 and 30 monosaccharide units. The polysaccharide component may be based on or derived from polysaccharide components of the polysaccharide capsule from many gram-positive and gram-negative bacterial pathogens such as *H. influenzae, N. meningitidis* and *S. pneumoniae.* Other bacteria from which polysaccharide components may be conjugated to the fusion proteins of the present invention include *Staphylococcus aureus, Klebsiella, Pseudomonas, Salmonella typhi, Pseudomonas aeruginosa, and Shigella dysenteriae.* Polysaccharide components suitable for use according to this aspect of the present invention include the Hib oligosaccharide, lipopolysaccharide from *Pseudomonas aeruginosa,* lipopolysaccharides from Salmonella, and the O-specific polysaccharide from *Shigella dysenteriae.*

Other polysaccharide components suitable for use in accordance with the present invention will be well-known to those skilled in the art.

Fragments of bacterial capsular polysaccharide may be produced by any suitable method, such as by acid hydrolysis or ultrasonic irradiation. Other methods of preparation of the polysaccharide components will be well known to those of skill in the art.

The polysaccharide is preferably a capsular polysaccharide derived from group B Streptococcus, or their equivalents.

The polysaccharide should preferably be coupled to the fusion protein by a covalent linkage. A particularly preferred method of coupling polysaccharide and protein is by reductive amination. Other methods include: activation of the polysaccharide with cyanogen bromide followed by reaction with adipic acid dihydrazide (spacer) and by conjugation to carboxide groups of carrier protein using soluble carbodiimides; functionalisation of the carrier protein with adipic acid dihydrazide followed by coupling to cyanogen bromide activated polysaccharides, chemical modification of both the carrier protein and the polysaccharide followed by their coupling.

The polysaccharide molecule may be coupled to the fusion protein by a spacer molecule, such as adipic acid. This spacer molecule can be used to facilitate the coupling of fusion protein to polysaccharide. After the coupling reaction has been performed, the conjugate may be purified by diafiltration or other known methods to remove unreacted protein or polysaccharide components.

If the polysaccharide is derived from a bacterial pathogen different from GBS, the conjugate may elicit immunity against two or more pathogens, e.g., multiple types of bacteria. This is a potentially important application of the immunogenic fusion protein.

A second aspect of the present invention concerns a nucleic acid molecule comprising a polynucleotide encoding the immunogenic fusion protein according to the first aspect of the present invention.

Such a nucleic acid molecule can be used in the manufacture of the immunogenic fusion protein directly, e.g., in a cell culture from which the immunogenic fusion protein is separated and obtained, or indirectly, e.g., in a subject in which the immunogenic fusion protein is to be expressed for immunization.

The nucleic acid molecule can e.g., be a DNA molecule or an RNA molecule. The RNA molecule may be a mRNA molecule.

The nucleic acid molecule may comprise, or preferably consist of, a first polynucleotide having at least 90%, such as at least 95%, at least 96%, at least 97%, at least 98% or at least 99%, or 100%, sequence identity to one of SEQ ID NOs: 1-6 fused directly, or via a linker polynucleotide sequence, to a second polynucleotide having at least 90%, such as at least 95%, at least 96%, at least 97%, at least 98% or at least 99%, or 100%, sequence identity to one of SEQ ID NOs: 8-13.

The linker polynucleotide sequence is optional and included if the immunogenic fusion protein encoded by the nucleic acid molecule is to have a linker provided between the first amino acid sequence part and the second amino acid sequence part. If the first polynucleotide is fused directly to the second polynucleotide, then there is no linker polynucleotide sequence present in the nucleic acid molecule. Each of the SEQ ID NOs: 1-6 and the SEQ ID NO:s 8-13 referred to above may be optionally truncated by removing the first three bases coding for the initial methionine (M) in the first amino acid sequence part, and the initial alanine (A) in the second amino acid sequence part of the immunogenic fusion protein encoded by the nucleic acid molecule.

The nucleic acid molecule may optionally comprise one or more stop codons.

The nucleic acid molecule may optionally comprise one or more start codons.

The nucleic acid molecule may be codon optimized for expression in a specific organism, such as *E. coli,* or *Cricetulus griseus* (Chinese Hamster Ovary) cells.

In a preferred embodiment of the nucleic acid molecule according to the second aspect of the present invention, the nucleic acid molecule has at least 90% sequence identity with the sequence of nucleotides shown in one of SEQ ID NOs: 15-20. Preferably the nucleic acid molecule has at least at least 95%, at least 96%, at least 97%, at least 98% or at least 99%, or 100%, sequence identity with the sequence of nucleotides shown in one of SEQ ID NOs: 15-20. Each of the SEQ ID NOs: 15-20 referred to in these preferred embodiments may be optionally truncated by removing the first three bases coding for the initial methionine (M) in the first amino acid sequence part of the immunogenic fusion protein encoded by the nucleic acid molecule. Further, each of the SEQ ID NOs: 15-20 referred to in these preferred embodiments may be optionally truncated by removing the last 1 to 6 bases of the two stop codons. This may for example be used when the nucleic acid molecule is to be used as a DNA or RNA vaccine - transcription and/or translation will cease at the end of the nucleic acid molecule.

The nucleic acid molecule may preferably consist of 1044 (without initial M) to 1047 bases (with initial M).

A third aspect of the present invention concerns a vector comprising the nucleic acid molecule according to the second aspect of the present invention.

A wide variety of expression host/vector combinations may be employed in expressing the nucleic acid molecule. Useful expression vectors for eukaryotic hosts include, for example, vectors comprising expression control sequences from SV40, bovine papilloma virus, adenovirus, adeno-associated virus, cytomegalovirus, and retroviruses. Useful expression vectors for bacterial hosts include bacterial plasmids, such as those from *E. coli,* including pBluescript, pGEX2T, pUC vectors, col E1, pCR1, pBR322, pMB9 and their derivatives, wider host range plasmids, such as RP4, phage DNAs, e.g., the numerous derivatives of phage lambda, e.g., lambda GT10 and lambda GT11, NM989, and other DNA phages, such as M13 and filamentous single stranded DNA phages. Useful expression vectors for yeast cells include the 2.mu. plasmid and derivatives thereof. Useful vectors for insect cells include pVL 941.

In addition, any of a wide variety of expression control sequences may be used in these vectors to express the nucleic acid molecules. Useful expression control sequences include the expression control sequences associated with structural genes of the foregoing expression vectors. Examples of useful expression control sequences include, for example, the early and late promoters of SV40 or adenovirus, the lac system, the trp system, the TAC or TRC system, the T3 and T7 promoters, the major operator and promoter regions of phage lambda, the control regions of fd coat protein, the promoter for 3-phosphoglycerate kinase or other glycolytic enzymes, the promoters of acid phosphatase, e.g., Pho5, the promoters of the yeast alpha-mating system and other constitutive and inducible promoter sequences known to control the expression of genes of prokaryotic or eukaryotic cells or their viruses, and various combinations thereof.

A fourth aspect of the present invention concerns a host cell expressing the immunogenic fusion protein according to the first aspect of the present invention or comprising the nucleic acid molecule according to the second aspect of the presentation invention or the vector according to the third aspect of the present invention.

The host cell may be a gram-negative bacterial cell, gram-positive bacterial cell, yeast cell, insect cell, animal cell, African green monkey cell, human cell, or plant cell. The host cell may be selected from the group consisting of *E. coli, Pseudomonas, Bacillus, Streptomyces, aspergillus, lactobacillus, shigella, salmonella, listeria, streptococcus, staphylococcus* and fungi. *E. coli* is however preferred.

Additionally, insect cells such as *Spodoptera frugiperda* (SF9), animal cells such as CHO and mouse cells, African green monkey cells such as COS 1, COS 7, BSC 1, BSC 40, and BMT 10, human cells, and plant cells in tissue culture, may be used. Preferred host organisms include bacteria such as *E. coli* and *B. subtilis,* and mammalian cells in tissue culture.

When a host cell is used to produce the immunogenic fusion protein, or used to produce the first and second amino acid sequence parts thereof, or used to produce the linker amino acid sequence part, then the produced amino acid sequence parts may be isolated from the microbial culture or cell culture and purified using any of a variety of conventional methods including: liquid chromatography such as normal or reversed phase, using HPLC, FPLC and the like; affinity chromatography (such as with inorganic ligands or monoclonal antibodies); ion exchange chromatography, size exclusion chromatography; immobilized metal chelate chromatography; gel electrophoresis; tangential flow filtration and the like. One of skill in the art may select the most appropriate isolation and purification techniques without departing from the scope of this invention.

In addition, the immunogenic fusion protein, the first and second amino acid sequence parts thereof, or the linker amino acid sequence part, may be generated by any of several chemical techniques. For example, they may be prepared using the solid-phase synthetic technique or they may be prepared by synthesis in solution. A

A fifth aspect of the present invention concerns a vaccine comprising one or more of the immunogenic fusion protein according to the first aspect of the present invention, the nucleic acid molecule according to the second aspect of the present invention, the vector according to the third aspect of the present invention, or the host cell according to the fourth aspect of the present invention.

The amount of each of the immunogenic fusion, the nucleic acid molecule, the vector, and/or the host cell is preferably a pharmaceutically effective amount, or an amount capable of eliciting protective immunity against group B Streptococcus, or an amount sufficient to elicit an immune response. Further, the amount may be such as to be sufficient to reduce and most preferably prevent a clinically significant deficit in the activity and response of the subject receiving the vaccine. Alternatively, the amount is sufficient to cause an improvement in a clinically significant condition in a host or subject.

As is appreciated by those skilled in the art, the amount of a compound may vary depending on its specific activity. Suitable dosage amounts may contain a predetermined quantity of active composition calculated to produce the desired therapeutic effect in association with the required diluents; i.e., carrier, or additive. Further, the dosage to be administered will vary depending on the active principle or principles to be used, the age, weight, etc. of the individual to be treated.

The vaccine is preferably capable of eliciting protective immunity against group B Streptococcus.

As noted in the figures, the immunogenic fusion protein elicits an immune response when administered to subjects. Such an immune response can also be obtained, indirectly, by administering the nucleic acid molecule either as a DNA nucleic acid molecule for later transcription and translation for expressing the immunogenic fusion protein, or as an RNA or mRNA nucleic acid molecule for translation and expression of the immunogenic fusion protein. The nucleic acid molecule may be provided in the vector for increased transcriptional and/or translational control, and the vector can in turn be provided in the host cell for further controlling and/or increasing the expression of the immunogenic fusion protein.

The vaccine may further comprise a pharmaceutically acceptable vehicle, and optionally an adjuvant. The term "pharmaceutical acceptable vehicle" is intended to mean any suitable acceptable excipient, adjuvants, carrier, diluent commonly used in pharmaceutical formulations.

The vaccine may encompass a vaccine composition.

The vaccine may additionally comprise other pharmacologically acceptable ingredients such as salts, buffers, immunoactive components, adjuvants (AlOH), wetting agents, emulsifying and suspending agents, or sweetening, flavouring, perfuming agents, or other substances which are desirable for improving the efficacy of the composition. A composition is said to be "pharmacologically acceptable" if its administration can be tolerated by a recipient individual.

Adjuvants are substances that can be used to specifically augment a specific immune response. Normally, the adjuvant and the composition are mixed prior to presentation to the immune system, or presented separately, but into the same site of the animal or human being immunized. Adjuvants can be loosely divided into several groups based upon their composition. These groups include oil adjuvants (for example, Freund's complete and incomplete), mineral salts for example, AlK (SO₄)₂, AlNa (SO₄)₂, AlNH₄ (SO₄), AlOH, silica, kaolin, and carbon), polynucleotides (for example, poly IC and poly AU acids), and certain natural substances (for example, wax D from *Mycobacterium tuberculosis,* as well as substances found in *Corynebacterium parvum,* or *Bordetella pertussis,* and members of the genus *Brucella.*

Alhydrogel (e.g., Aluminum hydroxide, AlOH) is an especially effective adjuvant for the immunogenic fusion protein according to the first aspect of the present invention and therefore especially useful in the vaccine according to the fifth aspect of the present invention.

Methods for the preparation and formulation of vaccines and vaccine compositions are well known to those skilled in the art. The choice of ingredients will for instance vary depending on the administration route of the composition. For example, compositions for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Carriers or occlusive dressings can be used to increase skin permeability and enhance antigen absorption. Liquid dosage forms for oral administration may generally comprise a liposome solution containing the liquid dosage form. Suitable forms for suspending liposomes include emulsions, suspensions, solutions, syrups, and elixirs containing inert diluents commonly used in the art, such as purified water.

The vaccine may be administered to humans or animals, including mammals and birds, such as rodents (mouse, rat, guinea pig, or rabbit); birds (turkey, hen or chicken); other farm animals (cow, horse, pig or piglet); pets (dog, cat and other pets); and humans. While many animals may be treated with the vaccine of the invention, a preferred individual for treatment is a human or commercially valuable animal and livestock such as fish, e.g., Tilapia, and camels.

The vaccine can be administered to an individual according to methods known in the art. Such methods comprise application e.g., parenterally, such as through all routes of injection into or through the skin: e.g., intramuscular, intravenous, intraperitoneal, intradermal, mucosal, submucosal, or subcutaneous. Also, they may be applied by topical application as a drop, spray, gel or ointment to the mucosal epithelium of the eye, nose, mouth, anus, or vagina, or onto the epidermis of the outer skin at any part of the body. Other possible routes of application are by spray, aerosol, or powder application through inhalation via the respiratory tract. In this last case the particle size that is used will determine how deep the particles will penetrate into the respiratory tract. Alternatively, application can be via the alimentary route, by combining with the food, feed or drinking water e.g., as a powder, a liquid, or tablet, or by administration directly into the mouth as a: liquid, a gel, a tablet, or a capsule, or to the anus as a suppository.

Many different techniques exist for the timing of the immunizations. It is possible to administer the vaccine or the components a-d more than once to increase the levels and diversities of expression of the immunoglobulin repertoire expressed by the immunized animal. Typically, if multiple immunizations are given, they may be given a week to three months apart, such as two weeks to two months apart, such as one to two months apart, or four weeks apart.

Additionally, one or maybe more booster injections of the vaccine or the components a-d may be administered. Preferably, booster injections may be administered at about 1 and 6 months after the initial injection or injections.

The vaccine according to the fifth aspect of the present invention may preferably comprise one or more additional immune active components. The additional immunoactive component may be an antigen, an immune enhancing substance, and/or a vaccine; either of these may comprise an adjuvant.

Accordingly, in a preferred embodiment of the vaccine according to the fifth aspect of the present invention, the vaccine additionally comprises one or more of:
a) a further immunogenic fusion protein comprising or consisting of an amino acid sequence having at least 90%, such as 95, 96, 97, 98 or 99 % sequence identity with the amino acid sequence shown in SEQ ID NO: 42;
b) a further nucleic acid molecule encoding the further immunogenic fusion protein, the further nucleic acid molecule preferably having at least 90% sequence identity with the sequence of nucleotides shown in one of SEQ ID NOs: 36-41;
c) a further vector comprising the further nucleic acid molecule; or
d) a further host cell expressing the further immunogenic fusion protein, or comprising the further vector.

As will be discussed further below, the further immunogenic fusion protein is derived from the N-terminal regions of the GBS surface proteins Alp1 and Alp2/3, as also disclosed in WO 2017/068112. As shown in the figures and discussed below, there is a synergistic effect when using a vaccine comprising both the immunogenic fusion protein and the further immunogenic fusion protein.

Further, by including one or more additional component of a-d, the vaccine can provide a broader coverage of protection against all Group B Streptococcus strains. Similar to as described above for the immunogenic fusion protein, the further immunogenic fusion protein may be expressed indirectly by the further nucleic acid molecule, further vector, and the further host cell.

The amount of each of a-d should preferably be a pharmaceutically effective amount, or an amount capable of eliciting protective immunity against group B Streptococcus, or an amount sufficient to elicit an immune response, or at least an amount sufficient to increase the general immune response to the vaccine.

As noted above, the further immunogenic fusion protein has at least 90%, such as 95, 96, 97, 98 or 99 % sequence identity with the amino acid sequence shown in SEQ ID NO: 42, which SEQ ID NO: 42 represents the following amino acid sequence:

MAEVISGSAATLNSALVKNVSGGKAYIDIYDVKNGKIDPLNLIVLTPSNYSA
NYYIKQGGRIFTSVNQLQTPGTATITYNILDENGNPYTKSDGQIDIVSLVTTV
YDTTELRNNINKVIENANDPKWSDDSRKDVLSKIEVIKNDIDNNPKTQSDID
NKIVEVNELEKLLVLPEFSTIPGSAATLNTSITKNIQNGNAYIDLYDVKNGLI
DPQNLIVLNPSSYSANYYIKQGAKYYSNPSEITTTGSATITFNILDETGNPHK
KADGQIDIVSVNLTIYDSTALRNRIDEVINNANDPKWSDGSRDEVLTGLEKI
KKDIDNNPKTQIDIDNKINEVNEIEKLLVVSL

Nucleic acid DNA molecules encoding the amino acid sequence of SEQ ID NO: 42, i.e., as encompassed by (b) are shown in SEQ ID NOs: 36, 38, and 40, where SEQ ID NO: 38 is codon optimized for expression in *E. coli,* and SEQ ID NO: 40 is codon optimized for expression in *Cricetulus griseus* (Chinese Hamster Ovary) cells.

Corresponding nucleic acid RNA molecules encoding the amino acid sequence of SEQ ID NO: 42 are shown in SEQ ID NOs: 37, 39, and 41, where SEQ ID NO: 39 is codon optimized for expression in *E. coli,* and SEQ ID NO: 41 is codon optimized for expression in *Cricetulus griseus* (Chinese Hamster Ovary) cells.

As mentioned above, the further immunogenic fusion protein is derived from the N-terminal regions of the GBS surface proteins Alp1 and Alp2/3.

The Alp1 protein is also known as epsilon protein and is a group B streptococcal alpha-protein-like protein.

The amino acid sequence of the N-terminal region of Alp1 is given by SEQ ID NO: 28:

MAEVISGSAATLNSALVKNVSGGKAYIDIYDVKNGKIDPLNLIVLTPSNYSA
NYYIKQGGRIFTSVNQLQTPGTATITYNILDENGNPYTKSDGQIDIVSLVTTV
YDTTELRNNINKVIENANDPKWSDDSRKDVLSKIEVIKNDIDNNPKTQSDID
NKIVEVNELEKLLVLP

Nucleic acid DNA molecules encoding the amino acid sequence of SEQ ID NO: 28 are shown in SEQ ID NOs: 22, 24, and 26, where SEQ ID NO: 24 is codon optimized for expression in *E. coli,* and SEQ ID NO: 26 is codon optimized for expression in *Cricetulus griseus* (Chinese Hamster Ovary) cells.

Corresponding nucleic acid RNA molecules encoding the amino acid sequence of SEQ ID NO: 28 are shown in SEQ ID NOs: 23, 25, and 27, where SEQ ID NO: 25 is codon optimized for expression in *E. coli,* and SEQ ID NO: 27 is codon optimized for expression in *Cricetulus griseus* (Chinese Hamster Ovary) cells.

The Alp2 protein is another alpha-protein-like-protein. Like the other members of the family, the Alp2 protein has an N-terminal domain and several repeated domains towards the C-terminus. The N-terminal region of Alp2 and Alp3 are identical. Alp3 is another alpha-protein-like-protein, also known as R28. It is very similar to the R28 protein also found in *S. pyrogenes.*

The amino acid sequence of the N-terminal region of Alp2 is given by SEQ ID NO: 35:

STIPGSAATLNTSITKNIQNGNAYIDLYDVKNGLIDPQNLIVLNPSSYSANYYI
KQGAKYYSNPSEITTTGSATITFNILDETGNPHKKADGQIDIVSVNLTIYDST
ALRNRIDEVINNANDPKWSDGSRDEVLTGLEKIKKDIDNNPKTQIDIDNKIN
EVNEIEKLLVVSL

Nucleic acid DNA molecules encoding the amino acid sequence of SEQ ID NO: 35 are shown in SEQ ID NOs: 29, 31, and 33, where SEQ ID NO: 31 is codon optimized for expression in *E. coli,* and SEQ ID NO: 33 is codon optimized for expression in *Cricetulus griseus* (Chinese Hamster Ovary) cells.

Corresponding nucleic acid RNA molecules encoding the amino acid sequence of SEQ ID NO: 35 are shown in SEQ ID NOs: 30, 32, and 34, where SEQ ID NO: 32 is codon optimized for expression in *E. coli,* and SEQ ID NO: 34 is codon optimized for expression in *Cricetulus griseus* (Chinese Hamster Ovary) cells.

SEQ ID NO: 42 thus correspond to SEQ ID NO: 28 (N-terminal of Alp1) fused via a linker amino acid sequence part consisting of the amino acids EF to SEQ ID NO: 35 (N-terminal of Alp2/3).

The second immunogenic fusion protein may thus comprise or consist of an amino acid sequence consisting of:
i. a further first amino acid sequence part having at least 90% sequence identity with the amino acid sequence shown in SEQ ID NO: 28;
ii. a further second amino acid sequence part having at least 90% sequence identity with the amino acid sequence shown in SEQ ID NO: 35; and optionally:
iii. a further linker amino acid sequence part consisting of 1 to 20 amino acids and separating the further first amino acid sequence part from the further second amino acid part,

In each of SEQ ID NO: 28 and 42, the initial M may be optional. Accordingly, in a preferred embodiment of the vaccine according to the fifth aspect of the present invention the vaccine comprises:
a) the immunogenic fusion protein according to the first aspect of the present invention, wherein the immunogenic fusion protein consists of the sequence of amino acids shown in SEQ ID NO: 21, or alternatively, wherein the immunogenic fusion protein consists of the amino acids 2-347 of SEQ ID NO: 21; and
b) a further immunogenic fusion protein comprising, preferably consisting of, the sequence of amino acids shown in SEQ ID NO: 42, or alternatively, comprising, preferably consisting of, the amino acids 2-347 of SEQ ID NO: 42.

In these embodiments there are preferably other no other proteins or amino acids sequences, different from the specified immunogenic fusion protein and the specified further immunogenic fusion protein, in the vaccine.

In a preferred embodiment of the vaccine according to the fifth aspect of the present invention the vaccine further comprises aluminum hydroxide as an adjuvant. In these embodiments the vaccine is preferably a pharmaceutical composition and further comprises a pharmaceutically acceptable excipient, diluent or carrier.

A sixth aspect of the present invention concerns the immunogenic fusion protein according to the first aspect of the present invention, the nucleic acid molecule according to the second aspect of the present invention, the vector according to the third aspect of the present invention, the host cell according to fourth aspect of the present invention, or the vaccine according to the fifth aspect of the present invention, for use in i) vaccination against group B Streptococcus infection, or ii) treating a group B Streptococcus infection.

The immunogenic fusion protein, the nucleic acid molecule, the vector, the host cell, or the vaccine may be used for such vaccination or treating by administering the immunogenic fusion protein, the nucleic acid molecule, the vector, the host cell, or the vaccine to a subject in need thereof.

A corresponding seventh aspect of the present invention concerns a method of i) vaccination against group B Streptococcus infection, or ii) treating a group B Streptococcus infection, by administrating the immunogenic fusion protein according to the first aspect of the present invention, the nucleic acid molecule according to the second aspect of the present invention, the vector according to the third aspect of the present invention, the host cell according to fourth aspect of the present invention, or the vaccine according to the fifth aspect of the present invention, to a subject in need thereof.

A corresponding eight aspect of the present invention concerns the use of the immunogenic fusion protein according to the first aspect of the present invention, the nucleic acid molecule according to the second aspect of the present invention, the vector according to the third aspect of the present invention, the host cell according to fourth aspect of the present invention for manufacturing a pharmaceutical composition or medicament, such as a vaccine, for i) vaccination against group B Streptococcus infection, or ii) treating a group B Streptococcus infection

The immunogenic fusion protein according to the first aspect of the present invention, the nucleic acid molecule according to the second aspect of the present invention, the vector according to the third aspect of the present invention, the host cell according to fourth aspect of the present invention, or the vaccine according to the fifth aspect of the present invention may thus be used in a method of preventing or treating an infection caused by a group B Streptococcus.

Maternal immunoprophylaxis with a vaccine, for protecting against infection to group B Streptococcus both in the mother and in the young infant, has long been proposed as a potential route.

Thus, in preferred embodiments of the sixth, seventh and eight aspects of the present invention, the immunogenic fusion protein according to the first aspect of the present invention, the nucleic acid molecule according to the second aspect of the present invention, the vector according to the third aspect of the present invention, the host cell according to fourth aspect of the present invention, or the vaccine according to the fifth aspect of the present invention is administered to a female human subject.

The administration is thus capable of conferring immunity to GBS infection to an unborn offspring of the human female. Further, the administration may also be made to a male if the male is older.

According to this embodiment, the immunogenic fusion protein, the nucleic acid molecule, the vector, the host cell, or the vaccine is administered to a non-pregnant female or to a pregnant female, under conditions of time and amount sufficient to cause the production of antibodies which serve to protect both the female and a fetus or newborn (via passive transfer of antibodies across the placenta).

The terms "preventing or treating" in its various grammatical forms in relation to the present invention refer to preventing, curing, reversing, attenuating, alleviating, ameliorating, inhibiting, minimizing, suppressing, or halting (1) the deleterious effects of a disorder associated with group B Streptococcus infection, (2) disorder progression, or (3) disorder causative agent (group B Streptococcus). Further, the terms "preventing or treating" are contemplated to include the creation of total or partial immunity of the individual to group B Streptococcus infection.

A ninth aspect of the present invention concerns a method for preventing or treating an infection caused by a group B Streptococcus which comprises administering to an individual in need thereof an effective amount of antibodies elicited from the exposure of a second individual to the immunogenic fusion protein, the nucleic acid molecule, the vector, the host cell, or the vaccine according to the first to fifth aspects of the present invention.

According to this aspect, resistance or immunity to group B Streptococcus is conferred to the individual by passive immunization, i.e., the elicited antisera is recovered and directly provided to a recipient suspected of having an infection caused by a group B Streptococcus. It is contemplated that such antisera could be administered to a pregnant female (at or prior to parturition), under conditions of time and amount sufficient so that the antisera would serve to protect either the fetus or newborn (via passive incorporation of the antibodies across the placenta). The vaccine or antisera of the present invention may, thus, be provided either prior to the onset of infection (so as to prevent or attenuate an anticipated infection) or after the initiation of an actual infection.

A tenth aspect of the present invention concerns a method of producing an immunogenic fusion protein comprising the steps of
- providing a host cell according to the fourth aspect of the present invention,
- multiplying the host cell,
- purifying the immunogenic fusion protein according to the first aspect of the present invention, and
- obtaining the immunogenic fusion protein.

Alternatively, the immunogenic fusion protein can be produced in a cell-free expression system. Such systems comprise all essential factors for expression from an appropriate recombinant nucleic acid, operably linked to a promoter that will function in that particular system.

### Example 1 - immunization of healthy women

Adult healthy women were immunized with an immunogenic fusion protein consisting of SEQ ID NO: 21 alone, or together with a further immunogenic fusion protein according to SEQ ID NO: 42, and with, or without, the adjuvant aluminum hydroxide.

Fig. 1 shows IgG fold increase obtained 29 days after immunization using one dose of either an immunogenic fusion protein according to the first aspect of the present invention alone, or in combination with a further immunogenic fusion protein as encompassed by the vaccine according to the fifth aspect of the present invention, at different dosages with or without the adjuvant aluminum hydroxide.

In Fig. 1, the first 7 results were obtained by administering an immunogenic fusion protein consisting of SEQ ID NO: 21 alone or together with the adjuvant aluminum hydroxide (0.5 mg).

As noted from Fig. 1, the use of Aluminum hydroxide is beneficial as it boosts the IgG fold increase of a 10 µg dose to a value similar to the IgG fold increase of a 50 µg dose. Similarly, a 50 µg dose together with aluminum hydroxide provides similar fold increase to a 250 µg dose.

Fig. 1 further shows that, when a vaccine comprising aluminum hydroxide and both an immunogenic fusion protein according to SEQ ID NO: 21 and a further immunogenic fusion protein according to SEQ ID NO: 42 is used, then doses of 25 µg of each of the immunogenic fusion proteins provide the same or higher fold increase compared to a dosage of 250 µg of the immunogenic fusion protein according to SEQ ID NO: 21. Using aluminum hydroxide and 50 µg of each of the immunogenic fusion proteins provided the highest fold increase at day 29.

The number of subjects (n) for each experiment is given in the figure.

Fig. 2 shows IgG fold increase obtained 57 days from immunization using a first dose and a second dose of either an immunogenic fusion protein according to the first aspect of the present invention alone, or in combination with a further immunogenic fusion protein as encompassed by the vaccine according to the fifth aspect of the present invention, at different dosages with or without an adjuvant.

In Fig. 2, the first 7 experiments are obtained by administering an immunogenic fusion protein consisting of SEQ ID NO: 21 alone or together with the adjuvant aluminum hydroxide (0.5 mg).

As noted from Fig. 2, the two doses of the immunogenic fusion protein consisting of SEQ ID NO: 21 at 50 µg and 100 µg together with Aluminum hydroxide provide a fold increase of about 100. However, even higher fold increases are seen when a vaccine comprising aluminum hydroxide and both an immunogenic fusion protein according to SEQ ID NO: 21 and a further immunogenic fusion protein according to SEQ ID NO: 42 is used, even though the dosage of each of the immunogenic fusion proteins is only 25 µg or 50 µg.

The number of subjects (n) for each experiment is given in the figure.

### Example 2 - immunization of female mice to protect pups

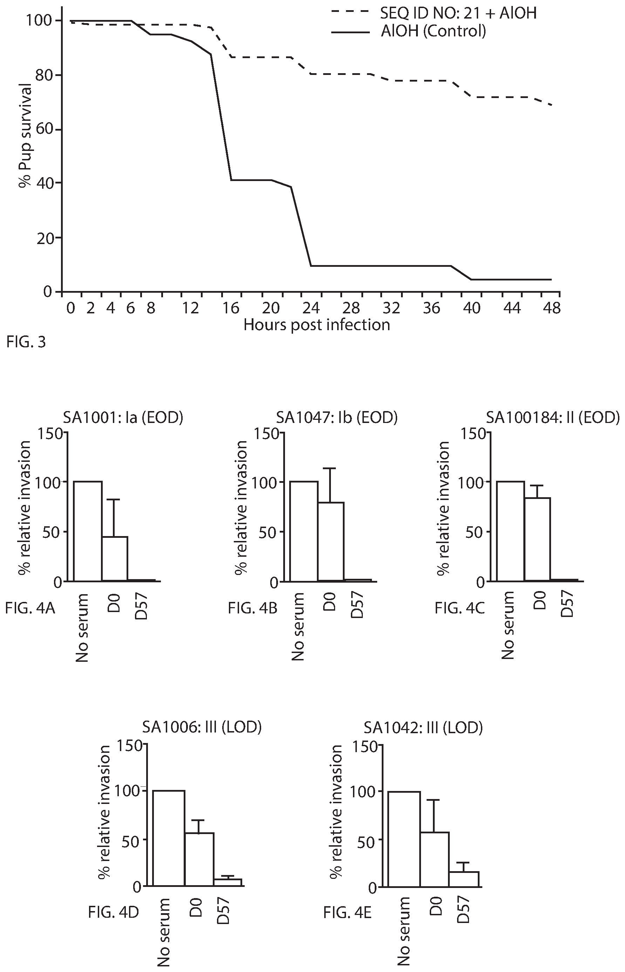

Fig. 3 shows survival of mice pups following neonatal bacterial infection with group B Streptococcus strain BM110 (8 x 10³ cfu). The pups were born from female mice having been immunized with either a) the immunogenic fusion protein of SEQ ID NO: 21 together with the adjuvant aluminum hydroxide (AlOH), b) or control (only the adjuvant aluminum hydroxide). As seen in the Fig. 3, mice pups born from mothers having been immunized with the immunogenic fusion protein according to SEQ ID NO: 21 (dashed line) had significantly higher survival rate throughout the experiment (0 - 48 hours post infection) than the mice pups born from mothers receiving the control (solid line). Accordingly, the immunogenic fusion protein of SEQ ID NO: 21, when administered to female mice, provided significant protection against GBS infection and death for mice pups born form those female mice. The immunogenic fusion protein thus provided a protective effect against GBS infection and death similar to as reported for mice in Panel C and E of Figure 2 of Lindahl et al, Nonimmunodominant Regions Are Effective As Building Blocks In A Streptococcal Fusion Protein Vaccine, Cell Host & Microbe 2, 427-434, December 2007.

### Example 3 - epithelial cell invasion study

Figs. 4A-4E show results obtained in a human epithelial cell invasion study using clinical isolates and antisera obtained from immunization using the immunogenic fusion of SEQ ID NO: 21. The respective bars show the relative percentage of epithelial cell invasion for the control (no serum), preimmune stage at day 0 (D0) and post immune stage at day 57 (D57). A good effect on inhibiting invasion of endothelial cells is shown. The immunogenic fusion protein thus provided an inhibitory effect on invasion of epithelial cells similar to as reported in Panel B of Figure 3 of Lindahl et al, Nonimmunodominant Regions Are Effective As Building Blocks In A Streptococcal Fusion Protein Vaccine, Cell Host & Microbe 2, 427-434, December 2007.

Accordingly, Figs 1 and 2 show that it is advantageous to administer the immunogenic fusion protein together with the further fusion protein, and that is also advantageous to use an adjuvant, e.g., aluminum hydroxide.

However, the immunogenic fusion protein elicited an immune response, e.g., IgG fold increase, also at low dosages (10 µg) and without adjuvant.

Further, Figs 3 and 4A-4E show that the immunogenic fusion protein provides effects similar to those seen for the immunogenic fusion protein disclosed in Lindahl et al, Nonimmunodominant Regions Are Effective As Building Blocks In A Streptococcal Fusion Protein Vaccine, Cell Host & Microbe 2, 427-434, December 2007 and WO 2008127179.

The dosage of each of the immunogenic fusion protein, and the further immunogenic fusion protein if administered, for humans may be in the range of 10 µg to 250 µg, such as from 10 µg to 100 µg, such as from 15 µg to 75 µg, preferably 25 µg to 75 µg, such as 25 µg to 50 µg.

The preferred dosage of each of the immunogenic fusion protein, and the further immunogenic fusion protein if administered, for humans, in the presence of an adjuvant such as aluminum hydroxide, is therefore within the range of 10 to 250 µg, preferably 10 to 150 µg, preferably 25 to 100 µg or 25 to 75 µg.

If the immunogenic fusion protein is administered together with the further immunogenic fusion protein, and in the presence of an adjuvant such as aluminum hydroxide, then the dosage of each fusion protein is preferably in the range of 15 µg to 75 µg, preferably 25 µg to 75 µg, such as 25 µg to 50 µg.

In the absence of an adjuvant such as aluminum hydroxide, then the dosage of each of the immunogenic fusion protein, and the further immunogenic fusion protein if administered, for humans, would be 10 to 1000 µg, preferably 50 to 500 µg, or preferably 100 to 250 µg.

If the immunogenic fusion protein, and the further immunogenic fusion protein if administered, are to administered indirectly by administering corresponding nucleic acid molecules, vectors or host cells, then the dosage of the corresponding nucleic acid molecules, vectors or host cells is preferably selected so as to obtain a similar immune response to what would have been obtained if the immunogenic fusion protein, and the further immunogenic fusion protein if administered, were administered directly to the subject.

Preferably the dosage of each of the immunogenic fusion protein, and the further immunogenic fusion protein if administered, is repeated. Accordingly, the immunogenic fusion protein, and the further immunogenic fusion protein if administered, are administered in a first dose, and repeated as a second dose some time after the first dose. The first dose and the second dose may be from a week to three months apart, such as two weeks to two months apart, such as one to two months apart, or four weeks apart.

### Example 4 - the fusion protein according to SEQ ID NO: 21 has reduced screening of conserved regions of the N-terminal of Rib

As noted initially, the present invention is based on further work on the fusion protein disclosed in WO 2008127179. This work identified the immunogenic fusion protein sequence, SEQ ID NO: 21, which is shorter than the immunogenic fusion protein disclosed in WO 2008127179 yet has a good effect and is suitable to use in a vaccine against GBS as shown in the above discussed figures.

The present example provides further insight into the difference between the shorter immunogenic fusion protein and the fusion protein of WO 2008127179. Specifically, the difference between the sequences is the 32 amino acid long leading sequence: GPLGSASVLIGISFLGGFTQGQFNISTDTVFA - SEQ ID NO: 43 which sequence is not found in SEQ ID NO: 21.

A computer simulation was performed on the sequence for the immunogenic fusion protein of WO 2008127179 including the leading sequence according to SEQ ID NO: 43. The simulation was based on molecular dynamics where each atom in a system is explicitly modeled. The forces acting on each atom by the neighboring atoms are also calculated and used to update the relative position of each atom at short time intervals, typically femtoseconds. The simulation produces a molecular trajectory where the whole conformation is written out at a specified time interval.

Specifically, a high-quality model of the full-length fusion protein of WO 2008127179 was prepared using Alphafold2, version v2.1.0, Jumper et al, Nature volume 596, pages583-589 (2021). The C-terminal domain, i.e. the N-terminal of AlpC was removed since it is far removed from the leading sequence, leaving the N-terminal rib domain including the 32 amino acid leading sequence (SEQ ID NO: 43) and totaling 204 amino acids. Gromacs 2021.5 was used to prepare the system as follows; the Alphafold2 model was solvated using water (gmx solvate) in a cubic unit cell, and ions were added to make the system neutral (gmx genion). The energy was minimized by keeping the heavy atoms constrained, followed by an equilibration step (pressure and temperature (at 300K)). The system was verified to be under a steady-state before proceeding with the main simulation as follows. The molecular movements of the global system was simulated for 100ns with a delta-time of 2fs for a total of 50 million steps, and the system conformation was stored every 5000 steps, resulting in a trajectory with 10000 models. MDanalysis v2.0.0 was used to analyze the global trajectory, verifying that the rib domain oscillates around a ground state, indicating that the molecule is stable under current conditions. The analysis was repeated three times (Sim1-3).

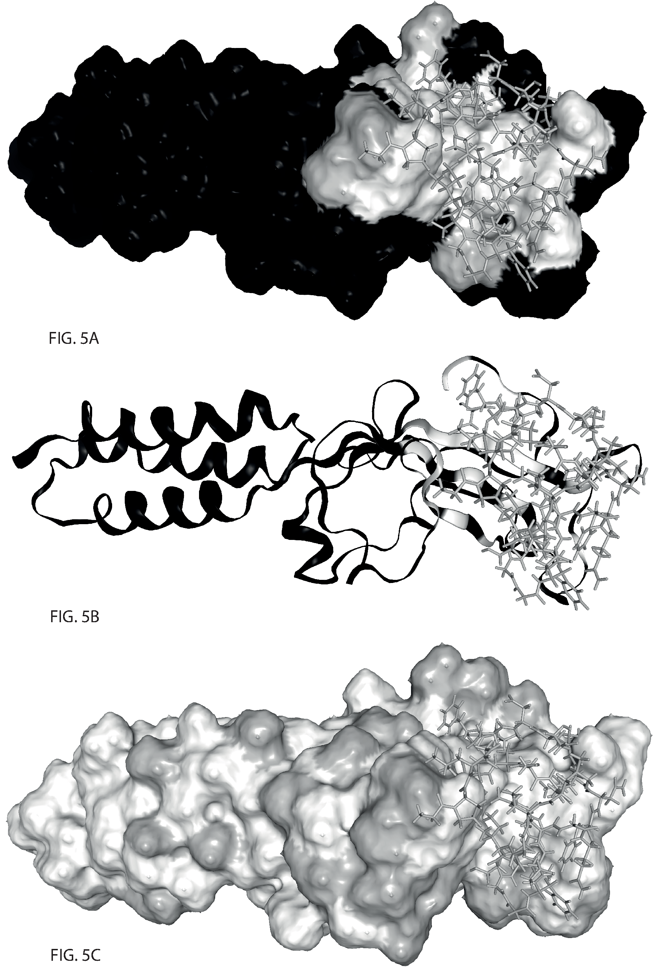

As a partial result, it was found that the leading sequence (amino acids 1-32) undergoes large conformational changes before finding a stable conformation after about 60 ns. Interactions between the leading sequence were calculated using Gromacs mindist (gmx mindist). To find tight and stable interactions between the leading sequence and the rib domain, the last 30ns of simulation (3000 models) were considered and interactions that occurred were calculated, on average in the three simulations, at least 30% of the time (interaction in 900 models or more). Interaction at this point was defined as a minimum distance between any atoms of the amino acids to be 0.3 nm or less. The results are presented in Table 1 below and shown in Figure 5A which shows the RibN terminal in surface representation with the leading sequence in ball and stick representation. The surface representation of the RibN terminal has been shaded from white corresponding to frequent contact (high fraction) to black corresponding to no or infrequent contact.

Fig. 5B shows an alternative representation of the RibN terminal (shown in cartoon representation) and leading sequence (shown in ball and stick representation). The cartoon representation of the RibN terminal has been shaded from white corresponding to frequent contact (high fraction) to black corresponding to no or infrequent contact.

Additionally, the amino acid conservation of the rib domain was calculated as follows; 144 complete Streptococcus agalactiae genomes were downloaded from PatricBRC, and among these, 95 rib domains were found using blastp 2.9.0+. The 95 sequences were aligned using Muscle v3.8.1551, and the normalized information content per amino acid was computed using logomaker 0.8. The conservation score is reported in the Conservation (Cons.) column in Table 1 below and shown in Fig. 5C which shows the RibN terminal in surface representation with the leading sequence in ball and stick representation. The surface representation of the RibN terminal has been shaded from white corresponding to high conservation score to black corresponding to no or a low conservation score.

**Table 1**

| **AA no in lead seq** | **Domain (RibN)** | **Sim.1** | **Sim.2** | **Sim.3** | **Avg.** | **Fraction** | **Cons.** |
|---|---|---|---|---|---|---|---|
| **1** | **91** | **2971** | **1** | **2** | **991** | **33.03%** | **0.60** |
| **2** | **93** | **3000** | **0** | **0** | **1000** | **33.32%** | **0.85** |
| **3** | 88 | 2553 | 1445 | 0 | 1333 | 44.41% | 0.75 |
| **3** | 91 | 2298 | 403 | 344 | 1015 | 33.82% | 0.60 |
| **3** | **93** | **2508** | **1872** | **0** | **1460** | **48.65%** | **0.85** |
| **4** | 92 | 0 | 0 | 2983 | 994 | 33.13% | 0.66 |
| **4** | **93** | **862** | **0** | **2955** | **1272** | **42.40%** | **0.85** |
| **5** | **93** | **2162** | **0** | **2746** | **1636** | **54.52%** | **0.85** |
| **10** | **111** | **0** | **0** | **2745** | **915** | **30.49%** | **0.75** |
| **11** | **86** | **0** | **0** | **2729** | **910** | **30.31%** | **0.76** |
| **12** | **93** | **2763** | **1436** | **4** | **1401** | **46.68%** | **0.85** |
| **13** | **86** | **2754** | **556** | **0** | **1103** | **36.77%** | **0.76** |
| **14** | 86 | 339 | 2931 | 0 | 1090 | 36.32% | 0.76 |
| **14** | 127 | 2824 | 0 | 0 | 941 | 31.37% | 0.75 |
| **14** | **129** | **2474** | **1177** | **0** | **1217** | **40.55%** | **0.98** |
| **15** | 84 | 1877 | 1665 | 0 | 1181 | 39.34% | 0.58 |
| **15** | 86 | 2 | 2980 | 0 | 994 | 33.12% | 0.76 |
| **15** | **111** | **2893** | **2992** | **0** | **1962** | **65.37%** | **0.75** |
| **15** | 113 | 2986 | 0 | 0 | 995 | 33.17% | 0.74 |
| **15** | 119 | 2971 | 0 | 0 | 990 | 33.00% | 0.98 |
| **16** | **84** | **2723** | **1504** | **0** | **1409** | **46.95%** | **0.58** |
| **18** | **119** | **0** | **2680** | **32** | **904** | **30.12%** | **0.98** |
| **23** | 119 | 2230 | 2339 | 1223 | 1931 | 64.33% | 0.98 |
| **23** | **127** | **2844** | **2907** | **2876** | **2876** | **95.82%** | **0.75** |
| **24** | 126 | 2989 | 2968 | 410 | 2122 | 70.72% | 1.00 |
| **24** | **127** | **2994** | **2998** | **2953** | **2982** | **99.36%** | **0.75** |
| **24** | 129 | 0 | 0 | 2739 | 913 | 30.42% | 0.98 |
| **25** | 126 | 2694 | 759 | 0 | 1151 | 38.35% | 1.00 |
| **25** | 127 | 2907 | 2858 | 0 | 1922 | 64.03% | 0.75 |
| **25** | **129** | **2818** | **2836** | **1203** | **2286** | **76.16%** | **0.98** |
| **26** | 109 | 0 | 0 | 2861 | 954 | 31.78% | 0.77 |
| **26** | 126 | 2622 | 871 | 0 | 1164 | 38.80% | 1.00 |
| **26** | **129** | **1002** | **1285** | **1504** | **1264** | **42.11%** | **0.98** |
| **27** | **93** | **0** | **2819** | **2968** | **1929** | **64.28%** | **0.85** |
| **28** | **88** | **0** | **2835** | **2985** | **1940** | **64.65%** | **0.75** |
| **29** | **131** | **2898** | **2893** | **0** | **1930** | **64.32%** | **0.77** |
| **30** | 130 | 2946 | 0 | 21 | 989 | 32.96% | 0.79 |
| **30** | **131** | **3001** | **2257** | **938** | **2065** | **68.82%** | **0.77** |
| **31** | 107 | 0 | 1856 | 1464 | 1107 | 36.88% | 0.75 |
| **31** | **131** | **2667** | **2987** | **2507** | **2720** | **90.65%** | **0.77** |
| **31** | 132 | 488 | 2558 | 0 | 1015 | 33.83% | 0.85 |
| **31** | 133 | 754 | 2425 | 25 | 1068 | 35.59% | 0.52 |
| **32** | **33** | **3001** | **3001** | **3001** | **3001** | **100.00 %** | **1.00** |
| **32** | 34 | 1222 | 1801 | 1731 | 1585 | 52.80% | 0.74 |
| **32** | 36 | 2987 | 2049 | 273 | 1770 | 58.97% | 0.98 |
| **32** | 130 | 2805 | 0 | 0 | 935 | 31.16% | 0.79 |
| **32** | 131 | 424 | 1753 | 2271 | 1483 | 49.41% | 0.77 |
| **32** | 132 | 173 | 1229 | 1871 | 1091 | 36.35% | 0.85 |

Table 1 shows stable interactions between the leading sequence (amino acids 1-32) and the rib domain (amino acids 33-204). Each amino acid pair between the leading sequence and the rib domain interacting at least 30% of the time in the last 30 ns of simulation is listed. The highest fraction for each leading sequence amino acid is bolded. The amino acid conservation was estimated and reported in the Cons. column.

As seen in table 1, four structure element pairs dominate the interactions; the first is the stretch of the leading sequence (29-32) closest to the domain that packs onto one of the main beta-sheets (129-133) of the Rib domain. The second element is between leading sequence amino acids 23-26 that interact with a loop-region (126-127) of the Rib domain that connects two beta-sheets. The third interaction is between the leading sequence amino acids 12-16 that interacts with several beta-sheets (84, 113, 127). Finally, the first part of the leading sequence(1-5) interacts with a loop (90-93) of the Rib domain.

These pairs of interactions involve parts of the N-terminal of Rib which have a high proportion of conserved amino acids, which high proportion shows that these parts are important for the intended purpose of the Rib protein, which is to facilitate infection of cells by the GBS bacteria.

Accordingly, the results in table 1 show that the 32 amino acid leading sequence of the RibN-AlpCN fusion protein of WO 2008127179, to a not insignificant degree, packs onto and interacts with these important parts of the Rib part of the fusion protein. The leading sequence thus timewise, i.e. at least 30% of the time, interacts with and decreases access to these parts. Accordingly, when used to elicit an immune response, the presence of the leading sequence decreases the immune system's access to these important parts of the Rib protein.

Conversely, the shorter immunogenic fusion protein according to SEQ ID NO: 21 does not include the leading sequence. There can thus not be any interactions that decrease the immune system's access to these important parts, and as a consequence a better immune response will be obtained.

Notably, the interactions between the leading sequence and the domains of Rib occur at different amino acids of the leading sequence. The shortening of the immunogenic fusion protein of WO 2008127179 will thus in each case provide a decreased interaction and better access for the immune system to all parts of the RibN domain of the immunogenic fusion protein. Accordingly, also a minor shortening, such as the removal of a lesser number of amino acids, of the leading sequence will provide better access for the immune system to the RibN domain of the immunogenic fusion protein.

As shown in Figures 5A-5C, the leading sequence interacts with one endcap of the N-terminal of Rib. The importance of this observation was investigated in Example 5 below.

### Example 5 - The conserved regions of the N-terminal of Rib screened by the leading sequence are involved when the Rib protein interacts with α1β1-integrin for adhesion and invasion of epithelial cells.

The significance of the conserved regions of the N-terminal of Rib found in Example 4 was studied. It was hypothesized that the conserved regions could be involved in facilitating adhesion to and invasion of epithelial cells, noting that Rib is a GBS surface protein and that antibodies towards RibN provides protection against GBS infection, including epithelial cell invasion.

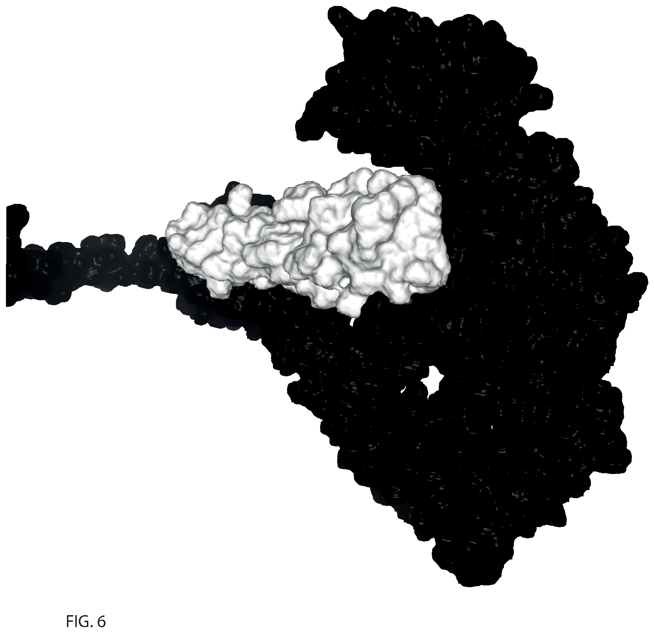

The alphafold structure for α1β1-integrin was obtained from the sequence referred to in Bolduc et al, Microbiology (2007), 153, 4039-4049, and interaction simulations between the N-terminal of Rib as contained in the fusion portion according to SEQ ID NO: 21 and α1β1-integrin were performed. Fig. 6 shows the result in which α1β1-integrin is shown in black surface representation and the N-terminal of Rib is shown in light grey surface representation. In Figure 6A, the N-terminal of Rib is shown in a similar orientation to Figure 5A-5C. Accordingly, while not shown in Figure 6, the leading sequence would be provided at the right endcap of the N-terminal of Rib. Comparison with Figure 5A-5C further showed that the conserved regions identified in Example 4 are involved in binding to α1β1-integrin. The results further showed that the N-terminal binds to domain 2 (von Willebrand factor, type A) and domain 3 of α1β1-integrin.

Examples 4 and 5 thus show that the leading sequence interacts with and screens a conserved region of the N-terminal of Rib, which conserved regions is involved with binding to α1β1-integrin, and thereby to epithelial cell adhesion and invasion.

Accordingly, immunization with a N-terminal region of Rib provided with the leading sequence will lead to a decreased exposure of these conserved regions due to the screening effect of the leading sequence. This will result in a less efficient immune system and/or antibody response against the conserved regions.

In contrast, if immunization is performed using an N-terminal or Rib in which the leading sequence (SEQ ID NO: 43) is removed or at least shortened, either using such a N-terminal alone or as part of the fusion protein of the first aspect of the present invention or SEQ ID NO: 21, then these conserved regions will be more accessible to the immune system. It will thus be easier for the immunized host to form antibodies against the conserved regions. A more efficient and complete neutralization of the conserved regions will increase the protection against epithelial cell adhesion and invasion via α1β1-integrin.

In summary, using a fusion protein according to the first aspect of the present invention, as exemplified by SEQ ID NO: 21, wherein the leading sequence (SEQ ID NO: 43) of the Rib constituent is removed or at least shortened, will provide improved protection against GBS, in particular by providing improved protection against adhesion and/or invasion of endothelial cells.

The invention is further illustrated by the following points.
1. An immunogenic fusion protein comprising or consisting of an amino acid sequence consisting of:
   i. a first amino acid sequence part consisting of 170 to 178 amino acids, preferably 174 to 175 amino acids, and having at least 90% sequence identity with the amino acid sequence shown in SEQ ID NO: 7;
   ii. a second amino acid sequence part consisting of 165 to 174 amino acids, preferably 169 to 170 amino acids, and having at least 90% sequence identity with the amino acid sequence shown in SEQ ID NO: 14; and optionally:
   iii. a linker amino acid sequence part consisting of 1 to 20 amino acids and separating the first amino acid sequence part from the second amino acid part,
   and wherein the immunogenic fusion protein preferably consists of 335 to 372 amino acids, preferably 343 to 353 amino acids, more preferably 343 to 347 amino acids.
2. The immunogenic fusion protein according to point 1, wherein:
   - the first amino acid sequence part comprises, or preferably consists of, amino acids 1-175 or 2-175 of the sequence shown in SEQ ID NO: 7, and
   - the second amino acid sequence part comprises, or preferably consists of, amino acids 1-170 or 2-170 of the sequence shown in SEQ DI NO: 14.
3. The immunogenic fusion protein according to any preceding point, wherein the amino acid sequence comprises the linker amino acid sequence part, and wherein the linker amino acid sequence part consists of 1 to 10 amino acids, preferably 2 to 5 amino acids, more preferably 2 amino acids, and most preferably the amino acid sequence EF.
4. The immunogenic fusion protein according to any preceding point, wherein the immunogenic fusion protein has at least 90% sequence identity to SEQ ID NO: 21.
5. The immunogenic fusion protein according to any preceding point, wherein the immunogenic fusion protein consists of the sequence of amino acids shown in SEQ ID NO: 21, or alternatively, wherein the immunogenic fusion protein consists of the amino acids 2-347 of SEQ ID NO: 21.
6. A nucleic acid molecule comprising a polynucleotide encoding the immunogenic fusion protein according to any of the proceeding points.
7. The nucleic acid molecule according to point 6, wherein the nucleic acid molecule has at least 90% sequence identity with the sequence of nucleotides shown in one of SEQ ID NOs: 15-20.
8. A vector comprising the nucleic acid molecule according to any of the points 6-7.
9. A host cell expressing the immunogenic fusion protein according to any of the points 1-5 or comprising the nucleic acid molecule according to any of the points 6-7 or the vector according to point 8.
10. A vaccine comprising one or more of the immunogenic fusion protein according to any of the points 1-5, the nucleic acid molecule according to any of the points 6-7, the vector according to point 8, or the host cell according to point 9.
11. The vaccine according to point 10, additionally comprising one or more of:
   a) a further immunogenic fusion protein comprising or consisting of an amino acid sequence having at least 90%, such as 95, 96, 97, 98 or 99 % sequence identity with the amino acid sequence shown in SEQ ID NO: 42;
   b) a further nucleic acid molecule encoding the further immunogenic fusion protein, the further nucleic acid molecule preferably having at least 90% sequence identity with the sequence of nucleotides shown in one of SEQ ID NOs: 36-41;
   c) a further vector comprising the further nucleic acid molecule; or
   d) a further host cell expressing the further immunogenic fusion protein, or comprising the further vector.
12. The vaccine according to point 11, wherein the vaccine comprises:
   a) the immunogenic fusion protein according to point 5; and
   b) a further immunogenic fusion protein comprising, preferably consisting of, the sequence of amino acids shown in SEQ ID NO: 42, or alternatively comprising, preferably consisting of, the amino acids 2-347 of SEQ ID NO: 42.
13. The vaccine according to any of the point 10-12, further comprising aluminum hydroxide as an adjuvant.
14. The immunogenic fusion protein according to any of the points 1-5, the nucleic acid molecule according to any of the points 6-7, the vector according to point 8, the host cell according to point 9, or the vaccine according to any of the points 10-13, for use in i) vaccination against group B Streptococcus infection, or ii) treating a group B Streptococcus infection.
15. The immunogenic fusion protein according to any of the points 1-5, the nucleic acid molecule according to any of the points 6-7, the vector according to point 8, the host cell according to point 9, or the vaccine according to any of the points 10-13, for use according to point 14, wherein the immunogenic fusion protein according to any of the points 1-5, the nucleic acid molecule according to any of the points 6-7, the vector according to point 8, the host cell according to point 9, or the vaccine according to any of the points 10-13, is administered to a female human subject.

## Claims

1. A vaccine comprising:
a) an immunogenic fusion protein consisting of amino acids 2-347 of SEQ ID NO. 21; and
b) a further immunogenic fusion protein consisting of amino acids 2-347 of SEQ ID NO: 42.

2. The vaccine according to claim 1, wherein a dose of the vaccine comprises 15 µg to 75 µg, preferably 25 µg to 75 µg, more preferably 25 µg to 50 µg, of each immunogenic fusion protein.

3. The vaccine according to claim 2, wherein a dose of the vaccine comprises 50 µg of each immunogenic fusion protein.

4. The vaccine according to any preceding claim, further comprising aluminum hydroxide as an adjuvant.

5. The vaccine according to claim 4, wherein a dose of the vaccine comprises 0.5 mg aluminium hydroxide.

6. The vaccine according to any preceding claim, wherein a dose of the vaccine comprises 50 µg of each immunogenic fusion protein and 0.5 mg aluminium hydroxide.

7. The vaccine according to any preceding claim for use as a medicament.

8. The vaccine according to any of the claims 1-6 for use in i) vaccination against group B Streptococcus infection or preventing a group B Streptococcus infection, or ii) treating a group B Streptococcus infection.

9. The vaccine for use according to any of the claims 7-8, wherein the vaccine is used at a dosage of 15 µg to 75 µg, preferably 25 µg to 75 µg, more preferably 25 µg to 50 µg, of each immunogenic fusion protein.

10. The vaccine for use according to claim 9, wherein the vaccine is used at a dosage of 50 µg of each immunogenic fusion protein.

11. The vaccine for use according to any of the claims 7-10, wherein the vaccine comprises aluminum hydroxide as an adjuvant.

12. The vaccine for use according to claim 11, wherein the vaccine is used at a dosage of 0.5 mg of aluminium hydroxide.

13. The vaccine for use according to claim 12, wherein the vaccine is used at a dosage of 50 µg of each immunogenic fusion protein and 0.5 mg of aluminium hydroxide.

14. The vaccine for use according to any of the claims 8-13, wherein vaccine is administered to a pregnant female human subject.

15. The vaccine for use according to any of the claims 8-13, wherein the vaccine is administered to an older human male.
